# EUROPÄISCHE PATENTSCHRIFT

(11) **EP 0 513 823 B1**
(45) Veröffentlichungstag und Bekanntmachung des Hinweises auf die Patenterteilung: **04.10.1995**
(21) Anmeldenummer: 92108249.1
(22) Anmeldetag: 15.05.1992
(51) Int. Cl.: C07C 19/08, C07C 17/20

(54) **Verfahren zur Herstellung von Pentafluorethan (R 125)**
Method for the production of pentafluoroethane (R125)
Procédé de fabrication de pentafluoroéthane (R125)

(30) Priorität: 17.05.1991 DE 4116209
(43) Veröffentlichungstag der Anmeldung: 19.11.1992
(73) Patentinhaber: HOECHST AKTIENGESELLSCHAFT, 65926 Frankfurt am Main (DE)
(72) Erfinder: Hoeveler, Martin, Dr., W-6233 Kelkheim (Taunus) (DE); Schnauber, Martin, Dr., W-6238 Hofheim am Taunus (DE); Schott, Martin, Dr., W-6374 Steinbach (DE)

(56) Entgegenhaltungen:
- EP-A- 0 130 532
- EP-A- 0 403 108
- EP-A- 0 417 680
- EP-A- 0 456 552
- FR-A- 2 433 500
- GB-A- 1 307 224
- PATENT ABSTRACTS OF JAPAN vol. 14, no. 453 (C-764)28. September 1990

## Beschreibung

Die vorliegende Erfindung betrifft ein Verfahren zur Herstellung von Pentafluorethan (R 125) durch Umsetzung von 1,1-Dichlor-2,2,2-trifluorethan (R 123) mit Fluorwasserstoff in der Gasphase. R 125 schädigt als chlorfreie Verbindung die Ozonschicht nicht und ist daher als Ersatzstoff beispielsweise für R 12 (Difluordichlormethan) in der Kältetechnik geeignet.

Die Herstellung von R 125 ist bereits bekannt. Die DE-OS 1 443 835 beschreibt ein Verfahren, bei welchem R 125 aus 1,1,1-Trichlor-2,2-difluorethan (R 122) durch Chlor-Fluor-Austausch mittels wasserfreiem Fluorwasserstoff bei 350°C an einem Chromoxyfluorid-Katalysator zugänglich ist. Jedoch werden nur Ausbeuten an R 125 von 42 % erzielt, neben 48 % nicht naher beschriebenen Nebenprodukten, bezogen auf eingesetztes R 122. Zudem ist ein mehr als neunfacher molarer Überschuß an Fluorwasserstoff erforderlich.

Die GB-PS 901 297 beschreibt die Herstellung von R 125 durch Additions- und Chlor-Fluor-Austauschreaktionen, ausgehend von Tetrachlorethen, mittels wasserfreiem Fluorwasserstoff in der Gasphase an einem Chromoxid-Katalysator. Auch nach diesem Verfahren sind 7 bis 9-fache molare Überschüsse an Fluorwasserstoff erforderlich. Es werden nur Ausbeuten an R 125 von ca. 10 %, bezogen auf eingesetztes Tetrachlorethen erzielt. Bei Einsatz der nur schwer zugänglichen und teuren Edukte 1,1-Dichlor-2,2-difluorethen bzw. Chlortrifluorethen werden Ausbeuten an R 125 bis ca. 29 % erzielt, unter Bildung von 16 - 22 % R 124 und weiteren, nicht näher bezeichneten Nebenkomponenten. Der Umsatz der eingesetzten Olefine liegt aber lediglich bei 62 %.

Aus der GB-PS 1 307 224 ist bereits die Herstellung von R 125 aus Tetrachlorethen oder 1,1-Dichlor-2,2,2-trifluorethan (R 123) durch Gasphasenfluorierung mit Fluorwasserstoff im molaren Verhältnis von 1:10 an einem Chromoxid-Katalysator bekannt. Die Umsetzungen verlaufen jedoch nicht selektiv. Beim Einsatz von Tetrachlorethen entsteht ein Gemisch aus 30 Vol.-% R 125, 20 Vol.-% R 124, 20 Vol.-% toxischem R 133a und 20 Vol.-% R 123. Wenn dagegen R 123 als Edukt benutzt wird, kann die Ausbeute an R 125 zwar auf 67 Vol.-%, neben 21 Vol-% R 124, erhöht werden, aber es entstehen gleichzeitig 2,5 Vol.-% Pentafluorchlorethan (R 115), welches mit R 125 ein Azeotrop bildet und somit von diesem nicht destillativ abgetrennt werden kann.

Gegenstand der Erfindung ist ein Verfahren zur Herstellung von Pentafluorethan (R 125) durch Reaktion von 1,1-Dichlor-2,2,2-trifluorethan (R 123) mit Fluorwasserstoff in der Gasphase, dadurch gekennzeichnet, daß ein Chrom und Magnesium enthaltender Katalysator verwendet wird, der dadurch erhältlich ist, daß man Chrom(III)-Hydroxid ausfallt, indem man 1 Mol eines wasserlöslichen Chrom(III)-Salzes mit mindestens 1,5 Mol Magnesiumhydroxid oder Magnesiumoxid in Gegenwart von Wasser umsetzt, die Reaktionsmischung in einen Teig Überführt, der Chromhydroxid und ein Magnesiumsalz enthält, und dann den Teig trocknet und bei Temperaturen von 20 bis 500°C mit Fluorwasserstoff behandelt.

Der Katalysator und seine Vorbehandlung (Aktivierung) wird in der EP-A-0 130 532 (= US-PS 4 547 483) beschrieben, auf deren Lehre hiermit ausdrücklich Bezug genommen wird ("incorporation by reference"). Aus EP-A-0 417 680 ist die Herstellung von 1,1,1,2-Tetrafluorethan aus 1,1,1-Trifluor-2-chlorethan und HF mit dem gleichen Katalysator bekannt.

Durch das erfindungsgemäße Verfahren läßt sich Überraschenderweise Pentafluorethan in hoher Ausbeute herstellen. Das dabei als Nebenprodukt anfallende R 124 läßt sich durch Rückführung in den Reaktor zu R 125 umsetzen. Das Verfahren hat den großen Vorteil, daß keine weiteren Nebenprodukte in wesentlichen Mengen entstehen.

Das Verfahren wird im allgemeinen nach Art üblicher Gasreaktionen an FestbettKatalysatoren durchgeführt, indem man das Gasgemisch aus R 123 und Fluorwasserstoff durch ein heizbares Reaktionsrohr leitet, das mit dem anspruchsgemäßen Katalysator gefüllt ist.

Das Reaktionsrohr wird vorzugsweise vertikal aufgestellt und besteht aus einem gegen Fluorwasserstoff hinreichend resistenten Werkstoff, wie Nickel oder Stahl.

Zur Durchführung des Verfahrens werden R 123 und Fluorwasserstoff im Gaszustand gemischt. Die Förderung der Edukte kann hierzu entweder (durch Erwärmen der Vorratsgefäße und Zuleitungen) gasförmig oder (durch Benutzung von Förderpumpen) flüssig erfolgen. Anschließend werden sie durch einen Vorerhitzer bzw. einen Verdampfer in den mit Katalysator gefüllten Reaktor geleitet. Die Ausgangssubstanzen werden vorteilhafterweise kontinuierlich zudosiert und in technischer Reinheit verwendet. R 123 ist beispielsweise nach dem Verfahren gemäß EP-A-0 349 298 zugänglich.

Bei Atmosphärendruck beträgt der Durchsatz an R 123 zWeckmäßigerweise 1 - 90 Liter (ca. 0,04 bis 4 mol), insbesondere 5 - 50 Liter (ca. 0,2 bis 2 mol), pro Liter Katalysator und Stunde. Bei höheren Drücken kann der Durchsatz an R 123 entsprechend höher liegen. Das Verfahren findet im allgemeinen bei Normaldruck oder geringem Überdruck statt, etwa 10⁻¹ bis 25 bar, vorzugsweise 1 bis 12 bar. Insbesondere zur Erzielung von höheren Raum-Zeit-Ausbeuten und zur Verringerung des Anteils olefinischer Nebenprodukte am Produktgas ist die Anwendung von Überdruck (2 bis 12 bar) vorzuziehen. Das Molverhältnis von HF zu R 123 beträgt im allgemeinen 2:1 bis 10:1, bevorzugt 2,5:1 bis 7:1.

Die Reaktion wird im allgemeinen bei Temperaturen von 200 bis 500°C, bevorzugt bei 320 bis 450°C durchgeführt.

Die Verweilzeit des Gasgemisches im Reaktor beträgt im allgemeinen 1 bis 60 s, bevorzugt 5 bis 30 s.

Der Umsatz des eingesetzten R 123 erreicht im allgemeinen Werte von ca. 85 %. Nahezu quantitative Umsätze können erreicht werden, wenn man die Anteile von R 123 und R 124 aus dem Produktgas abtrennt und mit dem Frischgas wieder in den Reaktor einbringt. Diese Kreisgas-Fahrweise bietet den Vorteil, die thermische Belastung des Produktes gering zu halten und trotzdem zu nahezu quantitativen Umsätzen und hohen Ausbeuten zu gelangen.

Die Aufarbeitung der Reaktionsprodukte kann nach zwei unterschiedlichen Methoden erfolgen:
1. Die gasförmigen Reaktionsprodukte werden durch Wasser oder wäßrige Alkalilauge geleitet, um den überschüssigen Fluorwasserstoff und den bei der Umsetzung entstandenen Chlorwasserstoff abzutrennen. Nach einer Trocknung, z.B. mit Calciumchlorid, wird das Rohgasgemisch nach Kondensation durch eine fraktionierte Destillation in eine R 125-Fraktion und das Restgasgemisch aus R 123 (Edukt) und R 124 (Zwischenprodukt) aufgetrennt, welches wieder in den Reaktor eingespeist werden kann.
2. Bei der anderen Aufarbeitungsweise werden die Gase nach Verlassen des Reaktors sofort einer fraktionierten Destillation unterworfen. Als Leichtsieder werden hierbei das Zielprodukt R 125 sowie der Chlorwasserstoff abgetrennt und das Restgasgemisch nach Versetzen mit der entsprechenden Menge Frischgas in den Reaktor zurückgefahren. Diese Methode hat den Vorteil, daß der überschüssige Fluorwasserstoff nicht ausgewaschen wird, sondern im Restgas verbleibt.

Die Vorteile des erfindungsgemäßen Verfahrens liegen im Erreichen hoher Selektivitäten (> 97 %) in der Summe von R 124 und R 125, wobei das gebildete R 124 in den Reaktor zurückgeführt und dort in weiteres R 125 umgewandelt werden kann. Nebenreaktionen, insbesondere Olefinbildung und Disproportionierungsreaktionen werden nahezu vollständig unterdrückt. Insbesondere entstehen weniger als 0,1 Vol.-% R 115, so daß Azeotropbildung vermieden wird. Dadurch wird auch die Aufarbeitung des Reaktionsprodukts entscheidend erleichtert.

Das Verfahren stellt einen erheblichen technischen Fortschritt dar, da es auf der Basis eines technisch erhältlichen Edukts (R 123) unter optimaler Ausnutzung der Ausgangsstoffe, ohne wesentliche Bildung von Isomerisierungs-, Disproportionierungs-, Oligo- oder Polymerisierungs- bzw. Fragmentierungsprodukten, die Herstellung von R 125 in hohen Ausbeuten ermöglicht. Damit verbunden ist eine einfache Aufarbeitung und ein leicht erreichbarer hoher Reinheitsgrad des Produktes.

Das erfindungsgemäße Verfahren wird durch die nachfolgenden Beispiele näher erläutert. Die angegebenen Prozente der GC-Analysen sind Flächenprozente.

### Versuchsbericht

### (Katalysatorherstellung gemäß EP-A 130 532 = US-PS 4 547 483)

200 g Cr(NO₃)₃ x 9 H₂O wurden in 1 l Wasser gelöst. Diese Lösung wurde zu einer Mischung von 500 g Magnesiumoxid und 240 g Graphit gegeben und die dabei entstehende pastöse Masse innig verknetet.

Anschließend wurde das pastöse Reaktionsprodukt zu Würfelförmlingen (0,5 cm Kantenlänge) granuliert und 16 Stunden bei 100°C getrocknet.

1 l (Schüttvolumen) der getrockneten Katalysatorkörper (= 600 g) wurden in einem Rohr aus Nickel oder VA-Stahl mit 5 cm lichter Weite und 100 cm Länge bei 200°C mit 15 mol Fluorwasserstoff behandelt. Die Dauer der Fluorwasserstoffbehandlung betrug ca. 6 Stunden. Dabei wurde das HF mit N₂ verdünnt. Der erhaltene Fluorierungskatalysator wies einen Chromgehalt von 2,3 Gew.-% auf.

### Beispiel 1

1,1-Dichlor-2,2,2-trifluorethan (R 123) und Fluorwasserstoff wurden im Molverhältnis von 1:5,7 über beheizte Leitungen einem Verdampfer zugeführt, vermischt und im gasförmigen Zustand über eine Schüttung von 0,5 Liter des gemäß Versuchsbericht hergestellten Katalysators in einem Rohrreaktor aus Nickel geleitet (Dimensionen des Reaktorrohres: 54 mm Durchmesser, ca. 1 m lang). Die Verweilzeit betrug 10,8 s. Der elektrisch beheizte Reaktor wurde bei einer Innentemperatur von 390°C gehalten. Die den Reaktor verlassenden gasförmigen Reaktionsprodukte wurden durch eine Wasserwäsche geleitet, die beheizt war, um das Auskondensieren von Reaktionsgasen zu verhindern. Die gasförmigen, nicht wasserlöslichen Reaktionsprodukte wurden mittels Calciumchlorid-Rohr getrocknet und gaschromatographisch analysiert.

Die Analyse ergab folgende Zusammensetzung:
56,6 % R 125 (Pentafluorethan)
25,6 % R 124 (2-Chlor-1,1,1,2-tetrafluorethan)
16,1 % R 123 (2,2-Dichlor-1,1,1-trifluorethan)

### Beispiel 2

In der in Beispiel 1 beschriebenen Apparatur wurde mit dem in Beispiel 1 beschriebenen Katalysator die Reaktion von R 123 mit HF bei 410°C Reaktorinnentemperatur, einem Molverhältnis von 1:5 und einer Verweilzeit von 10,3 s,aber ansonsten wie in Beispiel 1 durchgeführt. Die gaschromatographische Analyse des Produkts ergab folgendes:
58,3 % R 125
25,2 % R 124
14,1 % R 123

### Beispiel 3

Ein nach der Vorgehensweise in Beispiel 1 erhaltenes, HF-frei gewaschenes und getrocknetes Produktgasgemisch (Zusammensetzung laut gaschromatographischer Untersuchung: 23,0 % R 123; 31,9 % R 124; 43,2 % R 125) wurde erneut mit HF im Molverhältnis von ca. 1:5 gemischt und in den in Beispiel 1 beschriebenen Rohrreaktor geleitet. Der Versuch wurde bei 390°C und einer Verweilzeit von 9,5 s aber ansonsten wie in Beispiel 1 durchgeführt. Die gaschromatographische Analyse des Produkts ergab:
74,4 % R 125
17,7 % R 124
5,4 % R 123

## Patentansprüche

1. Verfahren zur Herstellung von Pentafluorethan (R 125) durch Reaktion von 1,1-Dichlor-2,2,2-trifluorethan (R 123) mit Fluorwasserstoff in der Gasphase, dadurch gekennzeichnet, daß ein Chrom und Magnesium enthaltender Katalysator verwendet wird, der dadurch erhältlich ist, daß man Chrom(III)-Hydroxid ausfällt, indem man 1 Mol eines wasserlöslichen Chrom(III)-Salzes mit mindestens 1,5 Mol Magnesiumhydroxid oder Magnesiumoxid in Gegenwart von Wasser umsetzt, die Reaktionsmischung in einen Teig überführt, der Chromhydroxid und ein Magnesiumsalz enthält, und dann den Teig trocknet und bei Temperaturen von 20 bis 500°C mit Fluorwasserstoff behandelt.

2. Verfahren nach Anspruch 1, dadurch gekennzeichnet, daß die Reaktion von R 123 mit Fluorwasserstoff im Temperaturbereich von 200-500°C durchgeführt wird.

3. Verfahren nach Anspruch 1, dadurch gekennzeichnet, daß die Reaktion von R 123 mit Fluorwasserstoff im Temperaturbereich von 320-450°C durchgeführt wird.

4. Verfahren nach einem der Ansprüche 1 bis 3, dadurch gekennzeichnet, daß die Reaktion von R 123 mit Fluorwasserstoff unter einem Druck von 1-12 bar durchgeführt wird.

5. Verfahren nach einem der Ansprüche 1 bis 4, dadurch gekennzeichnet, daß die Reaktion von R 123 mit Fluorwasserstoff bei einem Molverhältnis von 2 bis 10 Mol HF pro Mol R 123 durchgeführt wird.

## Claims

1. A process for the preparation of pentafluoroethane (R 125) by reacting 1,1-dichloro-2,2,2-trifluoroethane (R 123) with hydrogen fluoride in the gas phase, which comprises using a chromium- and magnesium-containing catalyst which is obtainable by precipitating chromium(III) hydroxide by reacting 1 mol of a water-soluble chromium (III) salt with at least 1.5 mol of magnesium hydroxide or magnesium oxide in the presence of water, converting the reaction mixture into a paste containing chromium hydroxide and a magnesium salt, and then drying the paste and treating the residue with hydrogen fluoride at temperatures of from 20 to 500°C.

2. The process as claimed in claim 1, wherein the reaction of R 123 with hydrogen fluoride is carried out in the temperature range of 200-500°C.

3. The process as claimed in claim 1, wherein the reaction of R 123 with hydrogen fluoride is carried out in the temperature range of 320-450°C.

4. The process as claimed in any one of claims 1 to 3, wherein the reaction of R 123 with hydrogen fluoride is carried out at a pressure of 1-12 bar.

5. The process as claimed in any one of claims 1 to 4, wherein the reaction of R 123 with hydrogen fluoride is carried out at a molar ratio of from 2 to 10 mol of HF per mole of R 123.

## Revendications

1. Procédé pour la préparation du pentafluoréthane (R 125) par réaction du 1,1-dichloro-2,2,2-trifluoréthane (R 123) avec du fluorure d'hydrogène en phase gazeuse, caractérisé en ce qu'on utilise un catalyseur contenant du chrome et du magnésium que l'on peut obtenir en précipitant l'hydroxyde de chrome(III), en faisant réagir 1 mole d'un sel de chrome(III) soluble dans l'eau avec au moins 1,5 mole d'hydroxyde de magnésium ou d'oxyde de magnésium en présence d'eau, en transformant le mélange réactionnel en pâte qui contient l'hydroxyde de chrome et un sel de magnésium, et en séchant ensuite la pâte et en traitant avec du fluorure d'hydrogène à des températures de 20 à 500°C.

2. Procédé selon la revendication 1, caractérisé en ce qu'on met en oeuvre la réaction de R 123 avec du fluorure d'hydrogène à une température de 200 à 500°C.

3. Procédé selon la revendication 1, caractérisé en ce qu'on met en oeuvre la réaction de R 123 avec du fluorure d'hydrogène dans un domaine de températures de 320 à 450°C.

4. Procédé selon l'une quelconque des revendications 1 à 3, caractérisé en ce qu'on met en oeuvre la réaction de R 123 avec le fluorure d'hydrogène sous une pression de 1 à 12 bar.

5. Procédé selon l'une quelconque des revendications 1 à 4, caractérisé en ce qu'on met en oeuvre la réaction de R 123 avec du fluorure d'hydrogène dans un rapport molaire de 2 à 10 moles de HF par mole de R 123.
